# EUROPEAN PATENT SPECIFICATION

(11) **EP 0 367 090 B1**
(45) Date of publication and mention of the grant of the patent: **12.01.1994**
(21) Application number: 89119819.4
(22) Date of filing: 25.10.1989
(51) Int. Cl.: G01N 33/94

(54) **Lyophilized MDM composition and method of making it**
Lyophilisiertes MDM enthaltende Zusammensetzung und Verfahren zu deren Herstellung
Composition lyophilisée de MDM et procédé pour sa préparation

(30) Priority: 01.11.1988 US 265749
(43) Date of publication of application: 09.05.1990
(73) Proprietor: SCHWARZ PHARMA AG, D-40789 Monheim (DE)
(72) Inventor: Wagle, Sudhakar S., Dr., 5600 W. Cty. Line Rd., Mequon, WN 53092 (US)

(56) References cited:
- EP-A- 0 278 243
- US-A- 4 183 910
- US-A- 4 316 882

## Description

### Field of the Invention

The present invention relates to compositions used to detect allergy or hypersensitivity to penicillins and methods of making them. More specifically, the present invention relates to a storage-stable, lyophilized MDM composition which can be reconstituted with water and which is used to detect allergy or hypersensitivity to penicillins. The invention also relates to the method of making this composition.

### Background of the Invention

Unfortunately, the useful antibiotic penicillin induces allergic reactions in patients with allergy or hypersensitivity to penicillin. Frequently, these reactions can be severe. In the worst cases, penicillin induces anaphylactic shock resulting in death due to cardiovascular collapse, ventricular arrythmia, or respiratory obstruction. In other less severe, yet still serious cases, penicillin induces nonfatal anaphylaxis and urticarial reactions, causing diffuse rash, difficulty in breathing, abdominal cramps, fainting, hypotension, or arrythmia.

The incidence of allergy or hypersensitivity to pencillins is significant. Physicians and researchers estimate that up to 10% of all patiens treated with penicillin will suffer an allergic reaction. Of these, 10% are life-threatening. Immediate reactions, or anaphylaxis, occur in .01% of all patients treated with penicillin. As to these, 9% of the allergic anaphylaxis reactions prove to be fatal. Even patients with no history of allergy or hypersensitivity to penicillin are at risk, for urticarial reactions have occurred in 4,5% of such patients.

Allergy or hypersensitivity to penicillin has been the principal factor which limits the usefulness of this otherwise beneficial medication. For many years, if a patient had a history of allergy or hypersensitivity to penicillin, doctors would label that patient as "allergic to penicillin" and would withhold penicillin from that patient for life. Instead of prescribing penicillin, doctors would treat such a patient with alternative antibiotics.

Despite the availability of many new antimicrobial agents, penicillin is still the drug of choice in some clinical situations. For example, alternative antibiotics are much less effective, and less tolerable, than penicillin for treating syphilis in pregnant woman. As another example, alternative antibiotics are not as well-suited as penicillin for treating bacterial endocarditis.

In the last two decades, physicians and researchers have discovered that allergy or hypersensitivity may have been overdiagnosed. If this is true, then many patients branded as "allergic to penicillin" could actually take this medication safely without experiencing a serious reaction.

Therefore, for all of these reasons, the medical world has long sought a reliable method for detecting allergy or hypersensitivity to penicillin.

The chemical mechanism by which penicillin triggers allergic reactions in patients with allergy or hypersensitivity, and the state of the art in "scratch" and "intradermal" skin tests for detecting such allergy or hypersensitivity, is extensively set forth in the literature. The following are intended only as representative citations which provide a useful technical background in the field of the invention:
1. Ressler C., Mendelson L.M.: Skin test for diagnosis of penicillin allergy - current status. Annals of Allergy 1987;59:167.
2. Levine B.B., Zolov D.M.: Prediction of penicillin allergy by immunological tests. J. Allergy 1969;43:231.
3. Adkinson N.F. Jr., Thompson W.L., Maddrey W.C., et al.: Routine use of penicillin skin testing as an inpatient service. N. Engl. J. Med. 1971;285:22.
4. Chandra R.K., Joglekar S.A., Tomas E.: Penicillin allergy. Arch. Dis. Child 1980;55:857.
5. Mendelson L.M., Ressler C., Rosen J.P., et al.: Routine elective penicillin allergy skin testing in children and adolescents: study of sensitization. J. Allergy Clin. Immunol. 1984;73:76.
6. Adkinson, N.F. Jr.: Private Communication, 1981.
7. Green G.R., Rosenblum A.H., Sweet L.C.: Evaluation of penicillin hypersensitivity: value of clinical history and skin-testing with penicilloylpolylysine and penicillin G. J.Allergy Clin. Immunol. 1988;60:339.
8. Sullivan T.J., Wedner H.J., Shatz G.S., et al.: Skin testing to detect penicillin allergy. J. Allergy Clin. Immunol. 1981;68:171.
9. Solley G.O., Gleich G.J., Van Dellen R.G.: Penicillin allergy: clinical experience with a battery of skin-test reagents. J. Allergy Clin. Immunol. 1982;69:238.
10. Warrington R.J., Simons F.E.R., Ho H.W., et al.: Diagnosis of penicillin allergy by skin testing: the Manitoba experience. Can. Med. Assoc. J. 1978;118:787.
11. Van Dellen R.G., Walsh W.E., Peters G.A., et al.: Differing patterns of wheal and flare skin reactivity in patients allergic to the penicillins. J. Allergy 1971;47:230.
12. Sogn D.D., Casale T.B., Condemi J.J., et al.: Interim results of the NIAID collaborative clinical trial of skin testing with major and minor penicillin derivatives in hospitalized adults. (abstract) J. Allergy Clin. Immunol. 1983;71 (suppl.):147.
13. Kerns D.L., Shira J.E., Go S. et al.: Ampicillin rash in children: relationship to penicillin allergy and infectious mononucleosis. Am. J. Dis. Child 1973;125:187.
14. Levine B.B:, Ovary Z.: Studies on the mechanism of the formation of the penicillin antigen. III. The N-(D-a-benzylpenicilloyl) group as an antigenic determinant responsible for hypersensitivity to penicillin G.J. Exp. Med. 1961;114:875.
15. Budd M.A., Parker C.W., Norden C.W.: Evaluation of intradermal skin tests in penicillin hypersensitivity. J. AM. Med. Assoc. 1964;190:203.
16. Levine B.B., Redmond A.P.: Minor haptenic determinant-specific reagents of penicillin hypersensitivity in man. Int. Arch Allergy 1969;35:445.
17. Levine B.B:, Redmond A.P., Fellner M.J., et al.: Penicillin allergy and the heterogeneous immune response of man to benzylpenicillin. J. Clin. Invest. 1966;45:1895.
18. Adkinson N.F. Jr., Spence M., Wheeler B.: Ranomized clinical trial of routine penicillin skin testing. (abstract) J. Allergy Clin. Immunol. 1984;73(suppl.):163.
19. Saxon, Beall, Rohr, Adelman: Am. Internal Med. 1987; 107:204-215

See also U.S. Patent Nos.4,316,882, 4,252,784, 4,228,147, and 4,183,910 issued to Levine; and U.S. Patent Nos. 3,979,508 and 3,867,365 issued to Stahmann and Wagle.

In prior studies, such as those cited above, it has been shown that when penicillin and its degradation products react with body tissues, immunogens are formed that are capable of provoking an immune response. The predominant tissue-bond moiety is benzylpenicilloyl. Because of its predominance, it is called the major antigenic determinant. Penicillin also forms additional antigenic determinants in smaller amounts. Because these determinants form in smaller quantities relative to the major determinant, they are called the minor antigenic determinants. "MDM" stands for "Minor Determinate Mixture".

The structures of the important penicillin determinants are shown below: 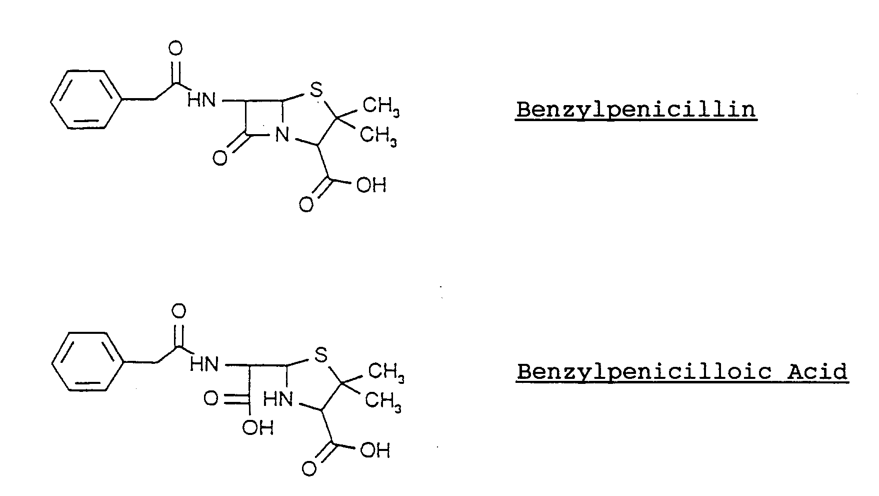 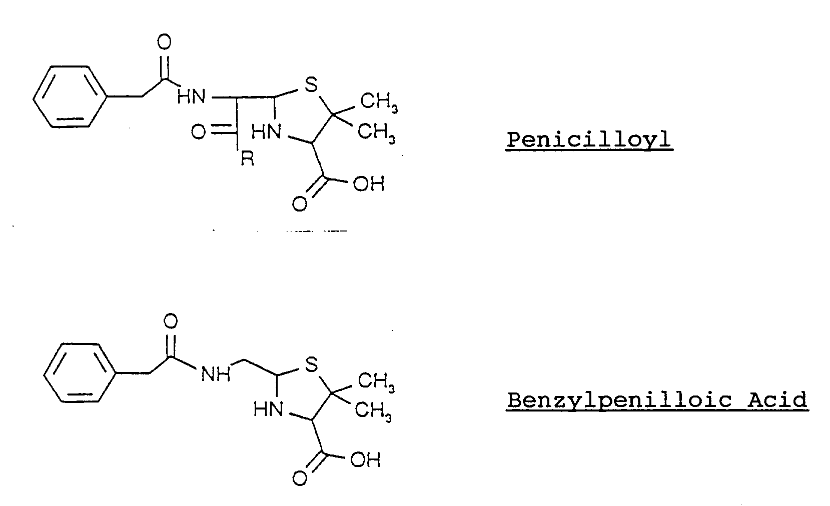
Because penicillin reacts with body tissues, patients who are treated with penicillin develop antibodies to the medication. Most patients produce IgG or IgM antibodies. These antibodies do not induce immediate hypersensitivity or allergic reaction.

Other patiens, however, develop IgE antibodies. It is the IgE antibodies which trigger an immediate allergic reaction to penicillin, and these reactions are the most dangerous. Thus, the presence of IgE antibodies signals allergy or hypersensitivity to penicillin in a patient.

Some IgE antibodies are specific for the major antigenic determinant, benzylpenicilloyl, and other IgE antibodies are specific for the MDM materials. While both major and minor determinants can provoke life-threatening allergic reactions, the MDM materials are associated with the majority of severe immediate reactions to penicillin.

Testing reagents comprising either the major determinant or the MDM materials are effective for detecting the presence of the IgE antibody. The most common testing reagent for detecting those IgE antibodies specific for the major determinant is penicilloyl-polylysine. This reagent is a synthetic conjugate of the benzylpenicilloyl determinant and a nonimmunogenic carrier.

Up to now, testing reagents for detecting those IgE antibodies specific for the MDM materials have comprised combinations of benzylpenicillin, benzylpenilloic acid, benzylpenicilloic acid, benzylpenicilloyl amines, and N-penicilloyl amines of an aliphatic amine or an α-amino acid. Recently, however, it has been learned that the first three of the listed materials are the relevant minor antigenic determinants. Because of this, it is believed that the benzylpenicilloyl amines offer no clinical advantage for skin testing.

Testing for allergy or hypersensitivity to penicillin using reagents for the major and minor determinants is safe and effective. Regardless of a patient's prior history of penicillin allergy, a negative test with both the major and minor determinants virtually rules out a serious allergic reaction to penicillin. Delayed reactions of a much less serious kind, though, are less completely excluded by a negative test. On the other hand, a positive test indicates a great likelihood that the patient is allergic or hypersensitive to penicillin. The penicillin challenge of such patients should not be undertaken.

Despite its efficacy, there continue to be problems with MDM compositions used for detecting allergy or hypersensitivity to penicillin. In particular, the MDM materials are unstable in solution due to degradation of the benzylpenicillin, the benzylpenilloic acid, and the benzylpenicilloic acid. In large part, this degradation is due to the epimerization of these materials. For example, the epimerization is so rapid that 34% of the benzylpenicilloic acid and 30% of the benzypenilloic acid epimerize in only one hour at about room temperature. As a result, only freshly prepared MDM compositions could be used with confidence until recently.

Because of the instability of the MDM materials, they have not been available commercially.

As disclosed in the prior art, certain MDM materials can be lyophilized in order to prepare compositions with an acceptable shelf-life. The Levine U.S. Patent No. 4,316,882 discloses a lyophilized MDM composition comprising benzylpenicillin, sodium benzylpenicilloate, and an N-penicilloyl-amine of an aliphatic amine or α-amino acid. In order to obtain an MDM composition with an acceptable shelf-life, these materials are lyophilized in single dose vials and are capable of reconstitution with an aqueous buffer just prior to use.

However, due to the inherent instability of the MDM materials in solution, the MDM compositions of Levine must be lyophilized at very cold temperatures and, therefore, at very slow rates. Thus, lyophilization is carried out for a total of 48 hours. During lyophilization, the temperature is kept at about -40°C for about 10 hours and is thereafter raised 2°C per hour until ambient temperature is reached.

### SUMMARY OF THE INVENTION

Accordingly, it is an object of our invention to provide a storage-stable MDM composition which can be lyophilized at a higher temperature and, therefore, at a faster rate than compositions known in the prior art.

A further object of our invention is to provide a storage-stable, lyophilized MDM composition which can be reconstituted with water to yield an isotonic solution of physiological pH which is used for detecting allergy or hypersensitivity to penicillins.

It is also an object of our invention to provide a method for making these compositions.

These and other objects are attained by the present invention as will be apparent from the following description.

In one aspect, the compositions of the invention comprise: benzylpenilloic acid; benzylpenicillin; benzylpenicilloic acid; an amount of an alkali metal salt effective to raise the melting point of the frozen MDM composition so that it can be lyophilized at a higher temperature under conditions sufficient to avoid meltback or collapse; a pH-buffering substance in a sufficient amount so that the pH-buffering substance and the alkali metal salt allow the lyophilized MDM composition to be reconstituted with water to yield an isotonic solution having a physiological pH; and an amount of water that is sufficiently low so that the degradation of the benzylpenilloic acid, the benzylpenicillin, and the benzylpenicilloic acid substantially is substantially prevented.

The compositions of the present invention are prepared according to a novel method. In the method of invention, an aqueous solution of benzylpenilloic acid, an alkali metal salt, and a pH-buffering substance is prepared. The benzylpenilloic acid in this solution is neutralized with a sufficient amount of aqueous sodium hydroxide so that the addition of the sodium hydroxide yields a concentration of sodium cations which effectively prevent epimerization, thus forming a stabilizing salt with the benzylpenilloic acid anions. The resulting solution is cooled to a temperature of from about 0°C to about 5°C.

While maintaining this solution at a temperature of from about 0°C to about 5°C, the following steps are performed: First, the benzylpenicillin and benzylpenicilloic acid are dissolved into the solution. The benzylpenicilloic acid in the solution is neutralized with an amount of aqueous sodium hydroxide so that the addition of the sodium hydroxide yields a concentration of sodium cations which effectively prevent epimerization, thus forming a stabilizing salt with the benzylpenicilloic acid anions. Next, the pH of the solution is adjusted to a physiological pH. This solution is then sterile filtered. This filtered solution is then dispensed into dehydrated containers.

After performing these steps in which the solution was maintained at a temperature of from about O°C to about 5°C, the solution in the dehydrated containers is frozen to a temperature of from about -45°C to about -15°C. After freezing, the frozen solution is lyophilized under conditions effective to avoid melt-back and collapse, whereby a lyophilized product is formed having a water content that is sufficiently low to substantially prevent the degradation of the benzylpenilloic acid, the benzylpenicillin, and the benzylpenicilloic acid.

The storage-stable, lyophilized MDM compositions of the present invention are useful for testing patients for allergy or hypersensitivity to penicillins according to test methods known in the art.

### DETAILED DESCRIPTION OF THE PRESENTLY PREFERRED EMBODIMENTS

The invention will be more fully appreciated in view of the following detailed description of the presently preferred embodiments.

### A. THE COMPOSITIONS OF THE INVENTION

Improved detection of allergy or hypersensitivity to penicillins can be achieved with the novel, storage-stable, lyophilized MDM compositions of the present invention. These compositions comprise benzylpenilloic acid, benzylpenicillin, benzylpenicilloic acid, an alkali metal salt, a pH-buffering substance, and a critically low amount of water. The compositions of the invention are characterized by a low level of epimerization of the MDM materials. For example, it is preferred that conditions that avoid epimerization be used so that at least about 94,5% of the benzylpenicilloic acid is the stereoisomer (5R,6R) - benzyl - D - penicilloic acid.

Generally, in order to obtain an MDM composition that is storage-stable, the MDM materials must be lyophilized. Lyophilization yields a product having a water content that is sufficiently low to substantially prevent the degradation of the MDM materials. To remove a sufficient amount of water, a MDM composition is lyophilized while frozen. Freezing is required because, while frozen, the composition exists in a crystalline form from which water can be readily extracted. If this crystalline structure is not maintained during lyophilization, then meltback and collapse occurs, and the MDM composition cannot be dried sufficiently to obtain a storage-stable composition having a low level of epimerization.

The compositions of the invention comprise an amount of an alkali metal salt effective to raise the melting point of the frozen MDM so that the composition can be lyophilized at a higher temperature under conditions sufficient to avoid meltback or collapse. As a result, the crystalline structure of the frozen lyophilate can be maintained over a higher temperature range than the frozen crystalline structure of a MDM composition that does not contain the alkali metal salt. Water is more readily removed from the frozen lyophilate at these higher temperatures. Hence, due to the presence of the salt, the MDM compositions of the present invention lyophilize at a higher temperature and, therefore, at a faster rate than compositions not utilizing such a salt.

This use of higher temperatures and a faster rate of lyophilization is advantageous because the chances of product collapse are decreased. Also, water is more easily removed from the MDM composition when it is lyophilized. In turn, the more efficient removal of water enhances the shelf-life of the lyophilized product.

Any alkali metal salt will work in the compositions of the invention, such as the alkali metal chlorides. Sodium chloride and potassium chloride are preferred, and potassium chloride is particularly preferred. Potassium chloride is particularly well-suited for use in the compositions of the invention because this salt provides a more ordered crystalline structure of the frozen MDM materials than other alkali metal salts provide. Additionally, potassium chloride increases the eutectic temperature of the frozen matrix more than other alkali metal salts. Even though the physiochemical reasons for this are not clearly understood, these superior lyophilizing properties of potassium chloride results in more rapid removal of water through the frozen matrix and allow lyophilization to occur at higher temperatures.

The composition of the invention comprise a pH-buffering substance in a sufficient amount so that the pH-buffering substance and the alkali metal salt allow the lyophilized MDM composition to be reconstituted with water to yield an isotonic solution having a physiological pH value of from about 7.3 to about 7.5. Sodium phosphate is preferred because of its superior pH-buffering properties. However, other pH-buffering substances can be used.

The compositions of the invention comprise a critically low amount of water. It is known that water causes the degradation of the benzylpenilloic acid, the benzylpenicillin, and the benzylpenicilloic acid. For example, in the presence of water, benzylpenicilloic acid undergoes epimerization to yield a mixture of four stereoisomers: the parent compound (5R,6R) - benzyl - D - penicilloic acid, (5S,6R) - benzyl - D - peni - cilloic acid, (5R,6S) - benzyl - D - penicilloic acid, and (5S,6S) - benzyl - D - penicilloic acid. The parent compound epimerizes completely in aqueous solution to the three other stereoisomers within 24 hours at about 4°C.

To illustrate, Example I provides data for the degradation of both benzylpenicillin and the parent compound of benzylpenicilloic acid in aqueous solution.

### EXAMPLE I - Stability of Benzylpenicilloic Acid and Benzylpenicillin in Buffered pH 7.3 Solution

| Compound | Temperature Degree C | Calculated 1st Order Rate Constant k hr⁻¹ | Hours to Achieve Concentration Loss of | |
|---|---|---|---|---|
| | | | 5 % | 10 % |
| MONOSODIUM (5R,6R)-BENZYL-D-PENICILLOATE TETRAHYDRATE | 0 | .0015035 | 34.1 | 70.1 |
| | 5 | .0033819 | 15.2 | 31.2 |
| | 10 | .0073926 | 6.9 | 14.3 |
| | 15 | .0157268 | 3.3 | 6.7 |
| | 20 | .0326057 | 1.6 | 3.2 |
| | 25 | .0659659 | .8 | 1.6 |
| PENICILLIN G POTASSIUM | 0 | .0000966 | 531.3 | 1091.2 |
| | 5 | .0001988 | 258.0 | 530.0 |
| | 10 | .0003989 | 128,6 | 264.1 |
| | 15 | .0007815 | 65.6 | 134.8 |
| | 20 | .0014963 | 34.3 | 70.4 |
| | 25 | .0028029 | 18.3 | 37.6 |

In this example, solutions of monosodium (5R,6R) - benzyl - D - penicilloate tetrahydrate and penicillin G potassium were tested using the HPLC assay method described in Vandino, W.A., et al.: J. Pharm. Sci. 1979; 68: 1316.

The result in this example show the hours required to achieve a concentration loss of 5% and 10% of the benzylpenicillin and the benzylpenicilloic acid over a temperature range from 0°C to 25°C.

Through degradation of the benzylpenilloic acid, the benzylpenicillin, and the benzylpenicilloic acid, water will severely truncate the shelf-life of any lyophilized MDM composition. Accordingly, the storage-stable, lyophilized MDM compositions of the invention comprise an amount of water that is sufficiently low so that the degradation of the benzylpenilloic acid, the benzylpenicillin, and the benzylpenicilloic acid is substantially prevented.

In order to maximize shelf-life, the compositions of the invention are most advantageously stored in dehydrated containers that have been dried so as to remove substantially all of the water. Thus stored, the compositions of the invention have about two-year stability at about 4°C and a one-year stability at about room temperature.

The preferred composition comprises: benzylpenilloic acid; from about 1.60 to about 1.85 moles of benzylpenicillin for each mole of benzylpenilloic acid; about 1.0 mole of benzylpenicilloic acid for each mole of benzylpenilloic acid; from about 9.38 to about 10.86 moles of an alkali metal salt for each mole of benzylpenilloic acid; and an amount of water that is sufficiently low so that degradation of the benzylpenilloic acid, the benzylpenicillin, and the benzylpenicilloic acid is substantially prevented.

### EXAMPLE II - Efficacy of Lyophilized Minor Determinant Mixture

The compositions of the invention are useful for testing patients for allergy or hypersensitivity to penicillins according to test methods known in the art.

Briefly, a lyophilized MDM composition prepared according to Example IV was stored at a temperature of about 4°C for about one week. Because of the enhanced stability of the MDM compositions of the present invention, the following results can also be achieved by using a lyophilized MDM composition prepared according to Example IV that has been stored at a temperature of from about 0°C to about 5°C for up to about two years, or that has been stored at about room temperature for up to about one year. Prior to testing, the lyophilized MDM was reconstituted with water to yield an isotonic solution having a physiological pH value of about 7.5. The concentration of ingredients in the reconstituted MDM solution is that shown in Example VII.

Skin testing was performed by the methods of scratch testing and intradermal injection testing. The scratch test involved superficial scratching of the skin and covering the scratch area with a drop of the MDM solution. If the results of the scratch test were negative, then the scratch test was followed by intradermal injection testing. The intradermal test was an injection of from about 0.010 ml to about 0.050 ml of the MDM solution. Clinical supplies were stored at a temperature of from about 0°C to about 5°C prior to use.

In conducting these tests, a positive skin test, which is diagnostic for penicillin allergy, was interpreted according to known guidelines for skin test reagents. When test results are positive, the penicillin challenge of such patients cannot be ethically undertaken. A negative test indicated no reaction at the skin test site.

In these tests all patients had a history of penicillin allergy. The results are shown in the following table.

| Skin Test Results | | | |
|---|---|---|---|
| Patient | Lyophilized MDM | Fresh MDM | Penicilloyl Polylysine |
| 1 | Negative | Negative | Negative |
| 2 | Negative | Negative | Negative |
| 3 | Positive | Positive | Negative |
| 4 | Positive | Positive | Negative |
| 5 | Positive | Positive | Positive |
| 6 | Positive | Positive | Positive |
| 7 | Negative | Negative | Positive |
| 8 | Negative | Negative | Positive |

The data illustrate the efficacy of the MDM compositions of the present invention. More specifically, these results show that, after prolonged storage, the compositions of the invention are as effective as is a freshly prepared MDM composition. The utility of the MDM compositions of the invention is highlighted by their ability to detect individuals with allergy or hypersensitivity whose allergy or hypersensitivity cannot be detected with penicilloyl polylysine.

### B. THE METHOD OF THE INVENTION

In the method of the invention, an aqueous solution is prepared comprising benzylpenilloic acid, an alkali metal salt, and a pH-buffering substance. Preferably, this solution comprises from about 9.38 to about 10.86 moles of the alkali metal salt for each mole of the benzylpenilloic acid, and about 1.0 mole of the pH-buffering substance for each mole of the benzylpenilloic acid.

The benzylpenilloic acid in this solution is neutralized with a sufficient amount of sodium hydroxide so that the addition of the sodium hydroxide yields a concentration of sodium cations which is effective to form a stabilizing and soluble salt with the benzylpenilloic acid anions. The rationale for this neutralization is that, in aqueous solution, the benzylpenilloic acid disassociates into a hydrogen cation and a benzylpenilloic acid anion, each ion carrying a single electrical charge. In this form, the benzylpenilloic acid is especially susceptible to degradation in aqueous solution. Accordingly, to stabilize the benzylpenilloic acid anion, these electrical charges must be neutralized. This is accomplished through the addition of the sodium hydroxide.

While other bases, such as potassium hydroxide, can also be used for neutralization, sodium hydroxide is particularly well-suited for use in the method of the invention. Generally, in aqueous solution, sodium hydroxide disassociates into a sodium cation and a hydroxide anion, each ion carrying a single electrical charge. Because each ion from the sodium hydroxide carries a single charge which is equal to, but opposite from, charges carried by the ions from the benzylpenilloic acid, sodium hydroxide is ionically compatible with the method of the invention. Further, the sodium cation, in particular, is critical for its characteristic of great ionic strength in aqueous solution.

When sodium hydroxide is added to the aqueous solution, the hydroxide anion neutralizes the hydrogen cation, and the sodium cation forms a stabilizing salt with the benzylpenilloic acid anion. After the addition of the sodium hydroxide, the concentration of the sodium cation in the solution should be from about 95% to about 110% of the concentration of the benzylpenilloic acid anion for the neutralization of the benzylpenilloic acid to be effective.

After neutralizing, the solution is cooled to a temperature of from about 0°C to about 5°C. While maintaining the solution at a temperature of from about 0°C to about 5°C, benzylpenicillin and benzylpenicilloic acid are dissolved in the solution. Preferably, the resulting solution comprises from about 1.60 to about 1.85 moles of benzylpenicillin for each mole of benzylpenilloic acid, and about 1.0 mole of benzylpenicilloic acid for each mole of benzylpenilloic acid. As was done with the benzylpenilloic acid, the benzylpenicilloic acid is neutralized by adding a sufficient amount of sodium hydroxide so that the addition of the sodium hydroxide yields a concentration of sodium cations which is effective to form a stabilizing salt with the benzylpenicilloic acid anions. After neutralization, the concentration of the sodium cations resulting from this addition of the sodium hydroxide should be from about 95% to about 110% of the concentration of the benzylpenicilloic acid anion for the neutralization to be effective.

While maintaining the solution at a temperature of from about 0°C to about 5°C, the pH is adjusted to a physiological pH value of from about 7.3 to about 7.5. This adjustment of the pH should be accomplished with aqueous solutions of either sodium hydroxide or hydrochloric acid. After adjusting the pH, the solution is sterile filtered while continuing to maintain a temperature of from about 0°C to about 5°C. After filtering, the solution is dispensed into dehydrated containers.

After performing these steps in which the solution is maintained at a temperature of from about 0°C to about 5°C, the solution in the dehydrated containers is frozen to a temperature of from about -45°C to about -15°C. Preferably, the solution should be frozen over a period of from about 20 to about 30 minutes.

After the dissolution of the benzylpenicilloic acid into the solution, the solution should be kept at a temperature of from about 0°C to about 5°C for a sufficiently brief length of time before proceeding on to said freezing in order to substantially prevent the epimerization of more than about 5.5% of the stereoisomer (5R,6R) - benzyl - D - penicilloic acid. Preventing this degree of epimerization can be accomplished if the solution is frozen within about 15 hours after the dissolution of the benzylpenicilloic acid.

After freezing, the frozen solution is lyophilized under conditions effective to avoid meltback and collapse, whereby a lyophilized product is formed having a water content that is sufficiently low to substantially prevent the degradation of the benzylpenilloic acid, the benzylpenicillin, and the benzylpenicilloic acid. Generally, many lyophilized systems can be used in the method of the invention. As an example, in the preferred method, the frozen solution is first lyophilized for from about 8 to about 13 hours at a temperature of from about -45°C to about -15°C under a vacuum of from about 15 to about 30 µm. While maintaining this vacuum, the frozen solution is further lyophilized for from about 7 to about 14 hours at a temperature of from about 0°C to about 5°C for a total drying time of from about 20 to about 22 hours.

After lyophilizing, the vacuum in the dehydrated containers is preferably broken using filtered, dried nitrogen. The dehydrated containers should be immediately sealed after breaking the vacuum. After sealing, the containers can be stored at a temperature of from about 0°C to about 5°C for prolonged periods of time up to about 2 years, or stored at about room temperature for prolonged periods of time for up to about one year.

The dehydrated containers used in the method of the invention can be any container that can be adequately dehydrated. For example, the lyophilized MDM compositions of the invention can be prepared and stored in sealed glass ampules or in rubber stoppered vials. In the case of rubber stoppered vials, the prior art fails to teach any special treatment of the rubber stoppers. We have observed, however, that autoclaving of the rubber stoppers for sterilization, i.e., sterilizing the rubber stoppers with heated steam under pressure, forces water into the rubber. If not dried at an elevated temperature, this water will leach from the rubber stoppers and enter the lyophilized product. If this happens, the water will cause the degradation of the benzylpenilloic acid, the benzylpenicillin, and the benyzlpenicilloic acid due to chemical reactions. To avoid this problem, the rubber stoppers and vials should be dried at a temperature of about 160°C for from about 1 to about 2 hours.

The lyophilized MDM compositions of the present invention should be storage-stable for up to about 2 years when stored at a temperature of from about 0°C to about 5°C and will also be storage-stable for up to about one year when stored at about room temperature. When reconstituted with water, the resulting aqueous solution of the MDM composition should have a physiological pH value of from about 7.3 to about 7.5.

### EXAMPLE III - Stability of MDM: Effect of Undried Rubber Stoppers

In this example, lyophilized MDM compositions prepared according to Example IV were placed in containers having either dried or undried rubber stoppers. The samples were reconstituted and tested using the HPLC assay method described in Vandino, W.A., et al.: J. Pharm. Sci. 1979; 68:1316. The results are shown in the following table.

| Sample¹ | Compound | Initial amount(%) | Amount Remaining² | Change |
|---|---|---|---|---|
| X1 Undried Stoppers | Penicillin | 100 | 68.4 | -31.6 |
| | Penicilloate | 100 | 115.0 | +15.0 |
| | Penilloate | 100 | 98.7 | - 1.3 |
| X5 Dried Stoppers | Penicillin | 100 | 97.5 | - 2.5 |
| | Penicilloate | 100 | 97.8 | - 2.2 |
| | Penilloate | 100 | 104.6 | + 4.6 |

| | | | | |
|---|---|---|---|---|
| 1. Rubber stoppers dried at 160°C for 1-2 hours and undried stoppers were air dried after autoclaving. | | | | |
| 2. X1 assayed after 4 months storage at room temperature. X5 assayed after 10 month storage at room temperature. | | | | |

These data show that, when undried rubber stoppers were used in preparing lyophilized MDM compositions, the benzylpenicillin, the benzylpenilloic acid, and the benzylpenicilloic acid underwent substantial degradation, thus reducing the shelf-life of the lyophilized MDM composition. After only 4 months, a 10-fold increase was found in the amount of benzylpenicillin lost when undried rubber stoppers were used as compared to when dried stoppers were used.

### EXAMPLE IV - Preparation of a Storage-Stable, Lyophilized MDM Composition Which Can Be Reconstituted With Water to Yield a Solution Which Is Used to Detect Allergy or Hypersensitivity to Penicillin

First, all glassware and utensils were depyrogenized before use. Filling and handling of open vials was done under aseptic, particulate free conditions.

Aqueous solutions of 0.1 M sodium hydroxide and 0.1 M hydrochloric acid were prepared. In preparing the sodium hydroxide solution, 1.00 gram of sodium hydroxide was weighed out and placed into a 250 ml volumetric flask. The sodium hydroxide in the flask was then diluted to the mark with Water for Injection. In preparing the hydrochloric acid solution, .83 ml of concentrated hydrochloric acid was pipetted into a 100 ml volumetric flask. The hydrochloric acid in this flask was then diluted to the mark with Water for Injection.

Next, 1.632 grams of benzylpenilloic acid monohydrate, 3.680 grams of potassium chloride, and .710 grams of dibasic, anhydrous sodium phosphate were placed into a 250 ml graduated cylinder. A small, magnetic stirring bar, 25 mm long by 9 mm in diameter, was also placed into the graduated cylinder to provide for mixing.

After this, 44.0 ml of the 0.1 M sodium hydroxide solution was added to the graduated cylinder in order to neutralize and dissolve the benzylpenilloic acid. The inside of the graduated cylinder was then rinsed with 10 ml of Water for Injection so that all solid ingredients were in solution. This solution was cooled in an ice bath to a temperature of from about 0°C to about 5°C.

While maintaining the solution in the graduated cylinder at a temperature of from about 0°C to about 5°C, 3.136 grams of potassium benzylpenicillin (penicillin G potassium) were added to the graduated cylinder. After adding the benzylpenicillin, the inside of the graduated cylinder was rinsed with 10 ml of Water for Injection. The solution was then mixed until the dissolution of the benzylpenicillin was complete.

Continuing to maintain the solution at a temperature of from about 0°C to about 5°C, 2.232 grams of monosodium benzylpenicilloate tetrahydrate was added to the solution in the graduated cylinder. After adding the benzylpenicilloic acid, the inside of the graduated cylinder was rinsed with 10 ml of Water for Injection. The solution was then stirred until dissolution of the benzylpenicilloic acid was complete. Following the dissolution of the benzylpenicilloic acid, 48.0 ml of 0.1 M sodium hydroxide solution was added in order to neutralize the benzylpenicilloic acid.

Next, while continuing to maintain the solution at a temperature at from about 0°C to about 5°C, the pH of the solution was adjusted to about 7.30 with 0.1 M sodium hydroxide or 0.1 M hydrochloric acid using a combination pH electrode rinsed with Water for Injection and removed from the graduated cylinder.

After this, while continuing to maintain the solution at a temperature at from about 0°C to about 5°C, the solution was diluted to final volume, yielding the solution for lyophilization. To accomplish this, the stirring bar was lifted from the solution using a magnet that was placed against the outside of the graduated cylinder. After removing the stirring bar, Water for Injection was added until the volume of the solution in the graduated cylinder was 200 ml. Following this dilution, the stirring bar was lowered back into the solution, followed by thorough mixing. Example V sets forth the concentrations of ingredients in the solution for lyophilization.

While continuing to maintain the solution for lyophilization at a temperature at from about 0°C to about 5°C, the solution was sterile filtered through a .2 µm, 47 mm diameter membrane in a laminar flow, high efficiency particulate air ("HEPA") hood. Use of the HEPA hood allowed for particulate free handling of the materials. The filter was first rinsed with about 30 ml of the solution. Some of this initial filtrate was collected in a vial for analysis by HPLC. After collecting this filtrate, from about 9 ml to about 10 ml portions of the filtered solution was dispensed into sterile serum vials using a sterile 10 ml graduated cylinder. Meanwhile, a bubble point membrane integrity test was performed on the filter according to the manufacturer's directions. The purpose of this integrity test was to ensure that there were no breaks, leaks, or other defects in the filter. Preferably, the sterile serum vials should be promptly sealed and placed into a -10°C freezer while performing the bubble point membrane integrity test.

Next, dehydrated containers were filled with the solution for lyophilization. The filling of the dehydrated containers was performed in a laminar flow, HEPA hood. To accomplish this, a vial of the solution from the -10°C freezer was thawed in an ice bath. While the solution in the vial was thawing, a number of 2 ml vials were placed into a metal pan. These 2 ml vials were then chilled by placing the pan on crushed ice. After thawing the solution for lyophilization to a temperature of from about 0°C to about 5°C, and without delay, from about 0.100 ml to about 0.105 ml portions of the solution for lyophilization were dispensed into the chilled 2 ml vials using a hand held pipettor having an autoclaved, polypropylene tip. Next, dried rubber stoppers were seated onto the 2 ml vials. The rubber stoppers had been dried at 160 °C for about 1 to 2 hours prior to use. After sealing the vials, between about 35 and 40 vials were transferred to crushed-ice-chilled Stoppering-Tainers (FTS Systems, Inc., Stone Ridge, NY) using a long forceps. The contents of each of the 2 ml vials are listed below in Example VI.

After filling the vials, the solution for lyophilization was frozen. To accomplish this, from about a -50°C to about a -40°C slush bath was prepared by cooling a mixture of 60% ethylene glycol and 40% water (percentages are by weight) with dry ice pellets. After preparing the slush bath, the solution for lyophilization contained in the 2 ml vials was frozen by immersing the Stoppering-Tainers in the slush bath for about 30 minutes.

After freezing, the lyophilization was performed. Lyophilization occured in two stages. First, in the primary drying stage, the frozen solution was lyophilized at a temperature of from about -45°C to about -15°C for about 8 to about 13 hours. As measured by a McLeod gauge, the vacuum during the primary drying stage was from about 15 to about 30 µm.

The primary drying stage was followed by a secondary drying stage. During this stage, the frozen solution was further lyophilized at a temperature of from about 0°C to about 5°C for from about 7 to about 14 hours under a vacuum of from about 15 to about 30 µm as measured by a McLeod gauge. The total drying time for both stages was from about 20 to about 22 hours. It is believed that the conditions for lyophilization may be varied provided that the product stability remains unchanged.

After lyophilizing, the vacuum tubing leading to the Stoppering-Tainers was clamped and the Stoppering-Tainers were removed from the system. Next, the vacuum in the Stoppering-Tainers was broken with nitrogen dried by a molecular sieve and filtered through a sterile 0.2 µm, 50 mm diameter filter unit (Millex-GF50, Millipore Corp., or the equivalent). Next, the 2 ml vials inside the Stoppering-Tainers were sealed by turning the Stoppering-Tainer handle counter-clockwise to drive the stoppering plate downward. After sealing the 2 ml vials, the vials were inspected. Any vial that had a powder residue in the neck was discarded. Such residue is undesirable, because the residue lowers the quality of the seal of the 2 ml vials. After capping and labeling the 2 ml vials, the vials were stored at a temperature of from about 0°C to about 5°C. The vials could also have been stored at about room temperature for up to about one year.

When the storage-stable, lyophilized MDM composition in one of the 2 ml vials was reconstituted to .25 ml with water, the resulting isotonic MDM solution had a physiological pH value of about 7.5. The concentration of ingredients in one of these reconstituted solutions is provided in Example VII.

### Example V - Concentration of Ingredients in the MDM Solution for Lyophilization

| Compound | Concentration |
|---|---|
| Benzylpenicilloic Acid | 25.0 mM |
| Benzylpenicillin | 42.1 mM |
| Benzylpenilloic Acid | 25.0 mM |
| Potassium Chloride | 246.8 mM |
| Sodium Phosphate | 25.0 mM |

### Example VI - Contents of Each Vial Containing Frozen Solution for Lyophilization

Each vial contains the following:

| | |
|---|---|
| Fill volume of solution for lyophilization | 0.10 ml |
| Potassium chloride | 1.840 mg |
| Dibasic, anhydrous sodium phosphate | 0.355 mg |
| Benzylpenilloic acid monohydrate | 0.816 mg |
| Potassium benzylpenicillin | 1.568 mg |
| Monosodium benzylpenicilloate Tetrahydrate | 1.116 mg |

### Example VII - Concentration of Ingredients in Reconstituted MDM solution

When reconstituted to 0.25 ml with water, the concentrations in each vial are:

| Compound | Concentration |
|---|---|
| Benzylpenilloic acid | 10.0 mM |
| Benzylpenicillin | 16.8 mM |
| Benzylpenicilloic acid | 10.0 mM |
| Potassium chloride | 98.7 mM |
| Sodium phosphate | 10.0 mM |

The resulting isotonic MDM solution will have a pH of about 7.5.

## Claims (Claims for the following Contracting State(s): AT, BE, CH, DE, FR, GB, IT, LI, LU, NL, SE)

1. A storage-stable, lyophilized Minor Determinant Mixture (MDM) composition which can be reconstituted with water to yield a solution which is used to detect allergy or hypersensitivity to penicillins, comprising:
(a) benzylpenilloic acid;
(b) from about 1.60 to about 1.85 moles of benzylpenicillin for each mole of benzylpenilloic acid;
(c) about 1.0 mole of benzylpenicilloic acid for each mole of benzylpenilloic acid wherein at least about 94.5% of the benzylpenicilloic acid is the stereoisomer (5R,6R) - benzyl - D - penicilloic acid
(d) from about 9.38 to about 10.86 moles of an alkali metal salt for each mole of benzylpenilloic acid;
(e) about 1.0 mole of a pH-buffering substance for each mole of benzylpenilloic acid; and
(f) an amount of water that is sufficiently low so that degradation of the benzylpenilloic acid, the benzylpenicillin, and the benzylpenicilloic acid is substantially prevented.

2. A storage-stable, lyophilized MDM composition as recited in claim 1, wherein said alkali metal salt is an alkali metal chloride.

3. A storage-stable, lyophilized MDM composition as recited in claim 1, wherein said alkali metal salt is potassium chloride.

4. A storage-stable, lyophilized MDM composition as recited in claim 1, wherein said alkali metal salt is sodium chloride.

5. A storage-stable, lyophilized MDM composition as recited in claim 1, wherein the pH-buffering substance is sodium phosphate.

6. A storage-stable, lyophilized MDM composition as recited in claim 1, said MDM composition being contained in a dehydrated container that has been dried so as to remove substantially all the water.

7. A process for preparing a storage-stable, lyophilized Minor Determinate Mixture (MDM) composition, wherein at least 94.5 % of the benzylpenicilloic acid is the stereoisomer (5R,6R)-benzyl-D-penicilloic acid, which can be reconstituted with water to yield a solution which is used to detect allergy or hypersensitivity to penicillins, which comprises the steps of:
(a) preparing an aqueous solution comprising:
(1) benzylpenilloic acid;
(2) from about 9.38 to about 10.86 moles of an alkali metal salt for each mole of the benzylpenilloic acid; and
(3) about 1.0 mole of a pH-buffering substance for each mole of benzylpenilloic acid;
(b) neutralizing the benzylpenicilloic acid in the solution of (a) with a sufficient amount of aqueous sodium hydroxide to yield a concentration of sodium cations which is effective to form a stabilizing salt with the benzylpenilloic acid anions;
(c) cooling the solution resulting from step (b) to a temperature of from about 0°C to about 5°C;
(d) performing the following steps while maintaining the solution of step (c) at a temperature of from about 0°C to about 5°C;
(i) dissolving from about 1.60 to about 1.85 moles of benzylpenicillin for each mole of benzylpenilloic acid in the solution resulting from step (c);
(ii) dissolving about 1.0 mole of benzylpenicilloic acid for each mole of benzylpenilloic acid in the solution resulting from step (d)(i);
(iii) neutralizing the benzylpenicilloic acid with an amount of aqueous sodium hydroxide sufficient to yield a concentration of sodium cations which is effective to form a stabilizing salt with the benzylpenicilloic acid anions;
(iv) adjusting the pH of the solution resulting from step (d)(iii) to a value of from about 7.3 to about 7.5; and
(v) sterile filtering the solution resulting from step (d)(iv);
(vi) dispensing the filtered solution resulting from step (d)(v) into dehydrated containers;
(e) freezing the solution in the dehydrated containers to a temperature of from about -45°C to about -15°C over about 30 minutes;
(f) lyophilizing the frozen solution, wherein during said lyophilizing, the frozen solution is first lyophilized for from about 8 to about 13 hours at a temperature of from about -45°C to about -15°C under a vacuum of from about 15 to about 30 µm, and is further lyophilized for from about 7 to about 14 hours at a temperature of from about 0°C to about 5°C under a vacuum of from about 15 to about 30 µm for a total drying time of from about 20 to about 22 hours.

8. A process for preparing a storage-stable, lyophilized MDM composition as recited in claim 7, wherein the alkali metal salt is an alkali metal chloride.

9. A process for preparing a storage-stable, lyophilized MDM composition as recited in claim 7, wherein the alkali metal salt is potassium chloride.

10. A process for preparing a storage-stable, lyophilized MDM composition as recited in claim 7, wherein the alkali metal salt is sodium chloride.

11. A process for preparing a storage-stable, lyophilized MDM composition as recited in claim 7, wherein the pH-buffering substance is sodium phosphate.

12. A process for preparing a storage-stable, lyophilized MDM composition as recited in claim 7, further comprising keeping the aqueous solution formed in step (d)(i) at a temperature of from about 0°C to about 5°C for a maximum time of 15 hours before proceeding on to step (e) to prevent the epimerization of more than about 5.5% of the stereoisomer (5R,6R) - benzyl - D - penicilloic acid.

13. A process for preparing a storage-stable, lyophilized MDM composition as recited in claim 7, further comprising the steps of breaking the vacuum in the dehydrated containers with filtered, dried nitrogen.

14. A process for preparing a storage-stable, lyophilized MDM composition as recited in claim 7, further comprising the steps of sealing the dehydrated containers immediately after breaking the vacuum.

15. A process for preparing a storage-stable, lyophilized MDM composition as recited in claim 7, wherein during said lyophilization, the frozen solution is first lyophilized for from about 8 to about 13 hours at a temperature of from about -45°C to about -15°C under a vacuum of from about 15 to about 30 µm, and is further lyophilized for from about 7 to about 14 hours at a temperature of from about 0°C to about 5°C under a vacuum of from about 15 to about 30 µm for a total drying time of from about 20 to 22 hours.

## Claims (Claims for the following Contracting State(s): ES, GR)

1. A process for preparing a storage-stable, lyophilized Minor Determinate Mixture (MDM) composition, wherein at least 94.5 % of the benzylpenicilloic acid is the stereoisomer (5R,6R)-benzyl-D-penicilloic acid, which can be reconstituted with water to yield a solution which is used to detect allergy or hypersensitivity to penicillins, which comprises the steps of:
(a) preparing an aqueous solution comprising:
(1) benzylpenilloic acid;
(2) from about 9.38 to about 10.86 moles of an alkali metal salt for each mole of the benzylpenilloic acid; and
(3) about 1.0 mole of a pH-buffering substance for each mole of benzylpenilloic acid;
(b) neutralizing the benzylpenicilloic acid in the solution of (a) with a sufficient amount of aqueous sodium hydroxide to yield a concentration of sodium cations which is effective to form a stabilizing salt with the benzylpenilloic acid anions;
(c) cooling the solution resulting from step (b) to a temperature of from about 0°C to about 5°C;
(d) performing the following steps while maintaining the solution of step (c) at a temperature of from about 0°C to about 5°C;
(i) dissolving from about 1.60 to about 1.85 moles of benzylpenicillin for each mole of benzylpenilloic acid in the solution resulting from step (c);
(ii) dissolving about 1.0 mole of benzylpenicilloic acid for each mole of benzylpenilloic acid in the solution resulting from step (d)(i);
(iii) neutralizing the benzylpenicilloic acid with an amount of aqueous sodium hydroxide sufficient to yield a concentration of sodium cations which is effective to form a stabilizing salt with the benzylpenicilloic acid anions;
(iv) adjusting the pH of the solution resulting from step (d)(iii) to a value of from about 7.3 to about 7.5; and
(v) sterile filtering the solution resulting from step (d)(iv);
(vi) dispensing the filtered solution resulting from step (d)(v) into dehydrated containers;
(e) freezing the solution in the dehydrated containers to a temperature of from about -45°C to about -15°C over about 30 minutes;
(f) lyophilizing the frozen solution, wherein during said lyophilizing, the frozen solution is first lyophilized for from about 8 to about 13 hours at a temperature of from about -45°C to about -15°C under a vacuum of from about 15 to about 30 µm, and is further lyophilized for from about 7 to about 14 hours at a temperature of from about 0°C to about 5°C under a vacuum of from about 15 to about 30 µm for a total drying time of from about 20 to about 22 hours.

2. A process for preparing a storage-stable, lyophilized MDM composition as recited in claim 1, wherein the alkali metal salt is an alkali metal chloride.

3. A process for preparing a storage-stable, lyophilized MDM composition as recited in claim 1, wherein the alkali metal salt is potassium chloride.

4. A process for preparing a storage-stable, lyophilized MDM composition as recited in claim 1, wherein the alkali metal salt is sodium chloride.

5. A process for preparing a storage-stable, lyophilized MDM composition as recited in claim 1, wherein the pH-buffering substance is sodium phosphate.

6. A process for preparing a storage-stable, lyophilized MDM composition as recited in claim 1, further comprising keeping the aqueous solution formed in step (d)(i) at a temperature of from about 0°C to about 5°C for a maximum time of 15 hours before proceeding on to step (e) to prevent the epimerization of more than about 5.5% of the stereoisomer (5R,6R) - benzyl - D - penicilloic acid.

7. A process for preparing a storage-stable, lyophilized MDM composition as recited in claim 1, further comprising the steps of breaking the vacuum in the dehydrated containers with filtered, dried nitrogen.

8. A process for preparing a storage-stable, lyophilized MDM composition as recited in claim 1, further comprising the steps of sealing the dehydrated containers immediately after breaking the vacuum.

9. A process for preparing a storage-stable, lyophilized MDM composition as recited in claim 1, wherein during said lyophilization, the frozen solution is first lyophilized for from about 8 to about 13 hours at a temperature of from about -45°C to about -15°C under a vacuum of from about 15 to about 30 µm, and is further lyophilized for from about 7 to about 14 hours at a temperature of from about 0°C to about 5°C under a vacuum of from about 15 to about 30 µm for a total drying time of from about 20 to 22 hours.

## Patentansprüche (Patentansprüche für folgende(n) Vertragsstaat(en): AT, BE, CH, DE, FR, GB, IT, LI, LU, NL, SE)

1. Lagerstabile, lyophilisierte Minor Determinant Mixture (MDM) Zusammensetzung, die mit Wasser zu einer Lösung rekonstituiert werden kann, die zur Detektion von Allergie oder Übersensibilität gegenüber Penicillinen verwendet wird, enthaltend:
(a) Benzylpenillosäure,
(b) im Bereich von 1,60 bis zu 1,85 Mole Benzylpenicillin für jedes Mol der Benzylpenillosäure,
(c) ungefähr 1,0 Mol Benzylpenicillosäure für jedes Mol der Benzylpenillosäure, worin wenigstens ungefähr 94,5 % der Benzylpenicillosäure das Stereoisomer (5R,6R)-benzyl-D-penicillosäure ist,
(d) im Bereich von 9,38 bis zu 10,86 Mole eines Alkalimetallsalzes für jedes Mol der Benzylpenillosäure,
(e) ungefähr 1,0 Mol einer pH-Puffer-Substanz für jedes Mol Benzylpenillosäure und
(f) eine Menge Wasser, die ausreichend gering ist, den Abbau der Benzylpenillosäure, des Benzylpenicillins und der Benzylpenicillosäure substantiell zu verhindern.

2. Lagerstabile, lyophilisierte MDM-Zubereitung nach Anspruch 1, in der das Alkalimetallsalz ein Alkalimetallchlorid ist.

3. Lagerstabile, lyophilisierte MDM-Zubereitung nach Anspruch 1, in der das Alkalimetallsalz Kaliumchlorid ist.

4. Lagerstabile, lyophilisierte MDM-Zubereitung nach Anspruch 1, in der das Alkalimetallsalz Natriumchlorid ist.

5. Lagerstabile, lyophilisierte MDM-Zubereitung nach Anspruch 1, in der die pH-Puffersubstanz Natriumphosphat ist.

6. Lagerstabile, lyophilisierte MDM-Zubereitung nach Anspruch 1, daß diese MDM-Zubereitung sich in einem dehytratisierten Behälter befindet, der derart getrocknet wurde, um das gesamte Wasser substantiell zu entfernen.

7. Verfahren zur Herstellung einer lagerstabilen lyophiliserten Minor Determinate Mixture (MDM) Zusammensetzung, in der wenigstens 94,5 % der Benzylpenicillosäure das Stereoisomer (5R,6R)-benzyl-D-penicillosäure ist, das mit Wasser zu einer Lösung rekonstituiert werden kann, die zur Detektion der Allergie oder Hypersensitivität gegenüber Penicillinen verwendet wird, das die folgenden Verfahrensschritte umfaßt:
(a) Herstellung einer wässrigen Lösung, enthaltend:
(1) Benzylpenillosäure;
(2) von 9.38 bis zu 10.86 Mole eines Alkalimetallsalzes für jedes Mol der Benzylpenillosäure, und
(3) 1,0 Mol einer pH-Puffersubstanz für jedes Mol der Benzylpenillosäure;
(b) Neutralisierung der Benzylpenicillosäure in der Lösung von (a) mit einer ausreichenden Menge wässrigen Natriumhydroxids bis zum Erreichen einer Konzentration an Natriumkationen, die ausreichend ist, ein stabiles Salz mit den Anionen der Benzylpenillosäure zu bilden;
(c) Kühlen der aus Schritt (b) erhaltenen Lösung auf eine Temperatur von 0°C bis zu 5°C;
(d) Ausführen der folgenden Schritte unter Vorhaltung der Lösung aus Schritt (c) bei einer Temperatur von 0°C bis zu 5°C;
(i) Auflösen von 1,60 bis zu 1,85 Mole Benzylpenicillin für jedes Mol Benzylpenillosäure in der aus Schritt (c) erhaltenen Lösung;
(ii) Auflösen eines 1.0 Moles Benzylpenicillosäure für jedes Mol Benzylpenillosäure in der aus Schritt (d) (i) erhaltenen Lösung;
(iii) Neutralisieren der Benzylpenicillosäure mit einer Menge wässrigen Natriumhydroxids, die ausreichend ist zum Erreichen einer Konzentration von Natriumkationen, die effektiv sind, ein stabilisiertes Salz mit den Benzylpenicillosäureanionen zu bilden;
(iv) Einstellen des pH der von Schritt (d) (iii) erhaltenen Lösung auf einen Wert von 7,3 auf bis zu 7,5; und
(v) Sterilfiltration der aus Schritt (d) (iv) erhaltenen Lösung;
(vi) Verbringen der filtrierten Lösung, die aus Schritt (d) (v) erhalten wurde in wasserfreie Behältnisse;
(e) Einfrieren der in den wasserfreien Behältnissen enthaltenen Lösung auf eine Temperatur von -45°C auf bis zu -15°C über bis zu 30 Minuten;
(f) Lyophilisieren der eingefrorenen Lösung, worin während des Lyophilisationsvorganges die gefrorene Lösung zuerst für 8 bis 13 Stunden bei einer Temperatur von -45°C bis zu -15°C unter einem Vakuum von 15 bis zu 30 µm lyophilisiert wird und weiter für 7 bis zu 14 Stunden bei einer Temperatur von 0°C bis zu 5°C unter einem Vakuum von 15 bis zu 30 µm bis zu einer Gesamttrocknungszeit von 20 bis zu 22 Stunden.

8. Verfahren zur Herstellung einer lagerstabilen, lyophilisierten MDM-Zusammensetzung nach Anspruch 7, in der das Alkalimetallsalz ein Alkalimetallchlorid ist.

9. Verahren zur Herstellung einer lagerstabilen, lyophilisierten MDM-Zusammensetzung nach Anspruch 7, in der das Alkalimetallsalz Natriumchlorid ist.

10. Verfahren zur Herstellung einer lagerstabilen, lyophilisierten MDM-Zusammensetzung nach Anspruch 7, in der das Alkalimetallsalz Natriumchlorid ist.

11. Verfahren zur Herstellung einer lagerstabilen, lyophilisierten MDM-Zusammensetzung nach Anspruch 7, in der die pH-Puffersubstanz Natriumphosphat ist.

12. Verfahren zur Herstellung einer lagerstabilen, lyophilisierten MDM-Zusammensetzung nach Anspruch 7, das weiter beinhaltet, daß die in Schritt (d) (i) gebildete wässrige Lösung bei einer Temperatur von 0°C bis zu 5°C für eine Höchstdauer von 15 Stunden gehalten wird, bevor mit Schritt (e) fortgefahren wir, um die Epimerisation von mehr als 5,5 % des Stereoisomers (5R,6R)-benzyl-D-penicillosäure zu verhindern.

13. Verfahren zur Herstellung einer lagerstabilen, lyophilisierten MDM-Zubereitung nach Anspruch 7, das weiterhin die Schritte beinhaltet, das Vakuum in den wasserfreien Behältnissen mit filtriertem, getrocknetem Stickstoff aufzuheben.

14. Verfahren zur Herstellung einer lagerstabilen, lyophilisierten MDM-Zusammensetzung nach Anspruch 7, das ferner die Schritte beinhaltet, die wasserfreien Behältnisse unmittelbar nach Aufhebung des Vakuums zu versiegeln.

15. Verfahren zur Herstellung einer lagerstabilen, lyophilisierten MDM-Zusammensetzung nach Anspruch 7, in dem während dieser Lyophilisation die gefrorene Lösung zuerst für 8 bis zu 13 Stunden bei einer Temperatur von -45°C bis zu -15°C unter einem Vakuum von 15 bis zu 30 µm lyophilisiert wird und dann für 7 bis zu 14 Stunden bei einer Temperatur von 0°C bis zu 5°C unter einem Vakuum von 15 bis zu 30 µm für eine Gesamttrocknungszeit von 20 bis zu 22 Stunden lyophilisiert wird.

## Patentansprüche (Patentansprüche für folgende(n) Vertragsstaat(en): ES, GR)

1. Verfahren zur Herstellung einer lagerstabilen lyophiliserten Minor Determinate Mixture (MDM) Zusammensetzung, in der wenigstens 94,5 % der Benzylpenicillosäure das Stereoisomer (5R,6R)-benzyl-D-penicillosäure ist, das mit Wasser zu einer Lösung rekonstituiert werden kann, die zur Detektion der Allergie oder Hypersensitivität gegenüber Penicillinen verwendet wird, das die folgenden Verfahrensschritte umfaßt:
(a) Herstellung einer wässrigen Lösung, enthaltend:
(1) Benzylpenillosäure;
(2) von 9.38 bis zu 10.86 Mol eines Alkalimetallsalzes für jedes Mol der Benzylpenillosäure, und
(3) 1,0 Mol einer pH-Puffersubstanz für jedes Mol der Benzylpenillosäure;
(b) Neutralisierung der Benzylpenicillosäure in der Lösung von (a) mit einer ausreichenden Menge wässrigen Natriumhydroxids bis zum Erreichen einer Konzentration an Natriumkationen, die ausreichend ist, ein stabiles Salz mit den Anionen der Benzylpenillosäure zu bilden;
(c) Kühlen der aus Schritt (b) erhaltenen Lösung auf eine Temperatur von 0°C bis zu 5°C;
(d) Ausführen der folgenden Schritte unter Vorhaltung der Lösung aus Schritt (c) bei einer Temperatur von 0°C bis zu 5°C;
(i) Auflösen von 1,60 bis zu 1,85 Mole Benzylpenicillin für jedes Mol Benzylpenillosäure in der aus Schritt (c) erhaltenen Lösung;
(ii) Auflösen eines 1,0 Moles Benzylpenicillosäure für jedes Mol Benzylpenillosäure in der aus Schritt (d) (i) erhaltenen Lösung;
(iii) Neutralisieren der Benzylpenicillosäure mit einer Menge wässrigen Natriumhydroxids, die ausreichend ist zum Erreichen einer Konzentration von Natriumkationen, die effektiv sind, ein stabilisiertes Salz mit den Benzylpenicillosäureanionen zu bilden;
(iv) Einstellen des pH der von Schritt (d) (iii) erhaltenen Lösung auf einen Wert von 7,3 auf bis zu 7,5; und
(v) Sterilfiltration der aus Schritt (d) (iv) erhaltenen Lösung;
(vi) Verbringen der filtrierten Lösung, die aus Schritt (d) (v) erhalten wurde in wasser-freie Behältnisse;
(e) Einfrieren der in den wasserfreien Behältnissen enthaltenen Lösung auf eine Temperatur von -45°C auf bis zu -15°C über bis zu 30 Minuten;
(f) Lyophilisieren der eingefrorenen Lösung, worin während des Lyophilisationsvorganges die gefrorene Lösung zuerst für 8 bis 13 Stunden bei einer Temperatur von -45°C bis zu -15°C unter einem Vakuum von 15 bis zu 30 µm lyophilisiert wird und weiter für 7 bis zu 14 Stunden bei einer Temperatur von 0°C bis zu 5°C unter einem Vakuum von 15 bis zu 30 µm bis zu einer Gesamttrocknungszeit von 20 bis zu 22 Stunden.

2. Verfahren zur Herstellung einer lagerstabilen, lyophilisierten MDM-Zusammensetzung nach Anspruch 1, in der das Alkalimetallsalz ein Alkalimetallchlorid ist.

3. Verahren zur Herstellung einer lagerstabilen, lyophilisierten MDM-Zusammensetzung nach Anspruch 1, in der das Alkalimetallsalz Natriumchlorid ist.

4. Verfahren zur Herstellung einer lagerstabilen, lyophilisierten MDM-Zusammensetzung nach Anspruch 1, in der das Alkalimetallsalz Natriumchlorid ist.

5. Verfahren zur Herstellung einer lagerstabilen, lyophilisierten MDM-Zusammensetzung nach Anspruch 1, in der die pH-Puffersubstanz Natriumphosphat ist.

6. Verfahren zur Herstellung einer lagerstabilen, lyophilisierten MDM-Zusammensetzung nach Anspruch 1, das weiter beinhaltet, daß die in Schritt (d) (i) gebildete wässrige Lösung bei einer Temperatur von 0°C bis zu 5°C für eine Höchstdauer von 15 Stunden gehalten wird, bevor mit Schritt (e) fortgefahren wir, um die Epimerisation von mehr als 5,5 % des Stereoisomers (5R,6R)-benzyl-D-penicillosäure zu verhindern.

7. Verfahren zur Herstellung einer lagerstabilen, lyophilisierten MDM-Zubereitung nach Anspruch 1, das weiterhin die Schritte beinhaltet, das Vakuum in den wasserfreien Behältnissen mit filtriertem, getrocknetem Stickstoff aufzuheben.

8. Verfahren zur Herstellung einer lagerstabilen, lyophilisierten MDM-Zusammensetzung nach Anspruch 1, das ferner die Schritte beinhaltet, die wasserfreien Behältnisse unmittelbar nach Aufhebung des Vakuums zu versiegeln.

9. Verfahren zur Herstellung einer lagerstabilen, lyophilisierten MDM-Zusammensetzung nach Anspruch 1, in dem während dieser Lyophilisation die gefrorene Lösung zuerst für 8 bis zu 13 Stunden bei einer Temperatur von -45°C bis zu -15°C unter einem Vakuum von 15 bis zu 30 µm lyophilisiert wird und dann für 7 bis zu 14 Stunden bei einer Temperatur von 0°C bis zu 5°C unter einem Vakuum von 15 bis zu 30 µm für eine Gesamttrocknungszeit von 20 bis zu 22 Stunden lyophilisiert wird.

## Revendications (Revendications pour l'(les) Etat(s) contractant(s) suivant(s): AT, BE, CH, DE, FR, GB, IT, LI, LU, NL, SE)

1. Une composition de Mélange Déterminant Mineur (MDM) lyophilisée et stable au stockage, laquelle peut être reconstituée avec de l'eau pour donner une solution destinée à détecter une aldergie ou une hypersensibidité aux pénicildines, et comprenant:
a) de l'acide benzylpénilloïque,
b) environ 1,60 à 1,85 modes de benzylpénicilline pour chaque mole d'acide benzylpénilloïque,
c) environ 1,0 mode d'acide benzylpénicilloïque pour chaque mode d'acide benzylpénilloïque, dont un minimum d'environ 94,5% de cet acide benzylpénicilloïque est constitué par l'acide stéréo-isomère (5R, 6R)-benzyl-D-pénicilloïque,
d) environ 9,38 à 10,86 modes d'un sel de métal alcalin pour chaque mode d'acide benzylpénilloïque,
e) environ 1,0 mode d'une substance tampon du pH pour chaque mode d'acide benzylpénilloïque, et
f) une quantité d'eau suffisamment basse pour inhiber substantiellement une dégradation de l'acide benzylpénilloïque, de la benzylpénicildine et de d'acide benzylpénicilloïque.

2. Une composition de MDM lyophilisée et stable au stockage selon la revendication 1, dans laquelle le sel de métal alcalin cité est un chlorure de métal alcalin.

3. Une composition de MDM lyophilisée et stable au stockage selon la revendication 1, dans laquelle le sel de métal alcalin cité est le chlorure de potassium.

4. Une composition de MDM lyophilisée et stable au stockage selon la revendication 1, dans laquelle le sel de métal alcalin cité est le chlorure de sodium.

5. Une composition de MDM lyophilisée et stable au stockage selon la revendication 1, dans laquelle la substance tampon du pH est le phosphate de sodium.

6. Une composition de MDM lyophilisée et stable au stockage selon la revendication 1, laquelle est contenue dans un récipient séché de telle sorte que toute eau en a été substantiellement éliminée.

7. Un procédé de préparation de la composition du mélange déterminant mineur (MDM), lyophilisée et stable au stockage, dans laquelle au moins 94,5% de l'acide benzylpénicilloïque sont constitués par de l'acide stéréoisomère (5R, 6R)-benzyl-D pénicilloïque, et laquelle peut être reconstituée avec de l'eau de façon à donner une solution destinée à détecter une allergie ou une hypersensibilité aux pénicillines, lequel procédé est caractérisé en ce qu'il consiste en les opérations suivantes:
a) la préparation d'une solution aqueuse comprenant:
1) de l'acide benzylpénilloïque,
2) environ 9,38 à 10,86 moles d'un sel de métal alcalin pour chaque mole d'acide benzylpénilloïque, et
3) environ 1,0 mole d'une substance tampon du pH pour chaque mole d'acide benzylpénilloïque,
b) la neutralisation de l'acide benzylpénicilloïque dans la solution de (a) avec une quantité d'hydroxyde de sodium aqueux suffisante pour donner une concentration de cations de sodium propre à la formation d'un sel stabilisateur avec les anions de l'acide benzylpénilloïque,
c) la réfrigération de la solution résultant de l'opération (b) à une température d'environ 0°C à 5°C,
d) le maintien constant de la solution résultant de (c) à une température d'environ 0°C à 5°C pendant toute la durée des opérations suivantes:
(i) la dissolution d'environ 1,60 à 1,85 moles de benzylpénicilline pour chaque mole d'acide benzylpénilloïque dans la solution résultant de l'opération (c),
(ii) la dissolution d'environ 1,0 mole d'acide benzylpénicilloïque pour chaque mole d'acide benzylpénilloïque dans la solution résultant de l'opération (d) (i),
(iii) la neutralisation de l'acide benzylpénicilloïque avec une quantité d'hydroxyde de sodium aqueux suffisante pour donner une concentration de cations de sodium propre à la formation d'un sel stabilisateur avec les anions d'acide benzylpénicilloïque,
(iv) l'ajustement du pH de la solution résultant de l'opération (d) (iii), à une valeur d'environ 7,3 à 7,5, et
(v) la filtration stérile de la solution résultant de l'opération (d) (iv),
vi) le transvasement de la solution filtrée résultant de l'opération (d) (v) dans des récipients déshydratés,
e) la congélation de la solution des récipients déshydratés à une température d'environ -45°C à -15°C pendant une durée approximative de 30 minutes,
f) la lyophilisation de la solution congelée, au cours de laquelle la solution congelée subit une première phase de lyophilisation durant environ 8 à 13 heures à une température d'environ -45°C à -15°C, sous un vide d'environ 15 à 30 µm, puis une seconde phase durant environ 7 à 14 heures à une température d'environ 0°C à 5°C, sous un vide d'environ 15 à 30 µm, la durée totale de dessiccation étant d'environ 20 à 22 heures.

8. Un procédé de préparation d'une composition de MDM lyophilisée et stable au stockage selon la revendication 7, caractérisé en ce que le sel de métal alcalin est un chlorure de métal alcalin.

9. Un procédé de préparation d'une composition de MDM lyophilisée et stable au stockage selon la revendication 7, caractérisé en ce que le sel de métal alcalin est le chlorure de potassium.

10. Un procédé de préparation d'une composition de MDM lyophilisée et stable au stockage selon la revendication 7, caractérisé en ce que le sel de métal alcalin est le chlorure de sodium.

11. Un procédé de préparation d'une composition de MDM lyophilisée et stable au stockage selon la revendication 7, caractérisé en ce que la substance tampon du pH est le phosphate de sodium.

12. Un procédé de préparation d'une composition de MDM lyophilisée et stable au stockage selon la revendication 7, caractérisé également en ce qu'il requiert le maintien constant de la solution aqueuse obtenue au cours de l'opération (d) (i) à une température d'environ 0°C à 5°C pendant une durée maximum de 15 heures avant de procéder à l'opération (e) en vue d'inhiber une épimérisation affectant plus de 5,5% de l'acide stéréo-isomère (5R,6R)-benzyl-D-pénicilloïque.

13. Un procédé de préparation d'une composition de MDM lyophilisée et stable au stockage selon la revendication 7, caractérisé en ce qu'il comprend également les opérations d'interruption du vide dans les récipients déshydratés à l'aide de nitrogène filtré et séché.

14. Un procédé de préparation d'une composition de MDM lyophilisée et stable au stockage selon la revendication 7, caractérisé en ce qu'il comprend également un bouchage hermétique des récipients déshydratés immédiatement après interruption du vide.

15. Un procédé de préparation d'une composition de MDM lyophilisée et stable au stockage selon la revendication 7, caractérisé en ce que, pendant la dite lyophilisation, la solution congelée subit une première phase de lyophilisation durant environ 8 à 13 heures à une température d'environ - 45°C à -15°C, sous un vide d'environ 15 à 30 µm, puis une seconde phase durant environ 7 à 14 heures à une température d'environ 0°C à 5°C, sous un vide d'environ 15 à 30 µm, la durée totale de dessiccation étant d'environ 20 à 22 heures.

## Revendications (Revendications pour l'(les) Etat(s) contractant(s) suivant(s): ES, GR)

1. Procédé de préparation d'une composition de mélange déterminant mineur (MDM), dans laquelle au moins 94,5% de l'acide benzylpénicilloïque sont constitués par de l'acide stéréo-isomère (5R,6R)-benzyl-D pénicilloïque, et laquelle peut être reconstituée avec de l'eau pour donner une solution destinée à détecter une allergie ou une hypersensibilité aux pénicillines, lequel procédé consiste en les opérations suivantes:
a) la préparation d'une solution aqueuse comprenant:
1) de l'acide benzylpénilloïque,
2) environ 9,38 à 10,86 moles d'un sel de métal alcalin pour chaque mole d'acide benzylpénilloïque, et
3) environ 1,0 mole d'une substance tampon du pH pour chaque mole d'acide benzylpénilloïque,
b) la neutralisation de l'acide benzylpénicilloïque dans la solution de (a) avec une quantité d'hydroxyde de sodium aqueux suffisante pour donner une concentration de cations de sodium propre à la formation d'un sel stabilisateur avec les anions d'acide benzylpénilloïque,
c) une réfrigération de la solution résultant de l'opération (b) à une température d'environ 0°C à 5°C,
d) le maintien constant de la solution résultant de l'opération (c) à une température d'environ 0°C à 5°C pendant toute la durée des opérations suivantes:
(i) dissolution d'environ 1,60 à 1,85 moles de benzylpénicilline pour chaque mole d'acide benzylpénilloïque de la solution résultant de l'opération (c),
(ii) dissolution d'environ 1,0 mole d'acide benzylpénicilloïque pour chaque mole d'acide benzylpénilloïque de la solution résultant de l'opération (d)(i),
(iii) neutralisation de l'acide benzylpénicilloïque à l'aide d'une quantité d'hydroxyde de sodium aqueux suffisante pour donner une concentration de cations propre à la formation d'un sel stabilisateur avec des anions d'acide benzylpénicilloïque,
(iv) ajustement du pH de la solution résultant de l'opération (d)(iii) à une valeur approximative de 7,3 à 7,5,
(v) filtrage stérile de la solution résultant de l'opération (d)(iv),
(vi) transvasement de la solution filtrée résultant de l'opération (d) (v) dans des récipients déshydratés,
e) la congélation de la solution des récipients déshydratés à une température d'environ -45°C à -15°C pendant environ 30 minutes,
f) la lyophilisation de la solution congelée, au cours de laquelle la solution congelée subit une première phase de lyophilisation durant environ 8 à 13 heures à une température d'environ -45°C à -15°C, sous un vide d'environ 15 à 30 µm, puis une seconde phase durant environ 7 à 8 heures à une température d'environ 0°C à 5°C, sous un vide d'environ 15 à 30 µm, la durée totale de la dessiccation étant d'environ 20 à 22 heures.

2. Procédé de préparation d'une composition de MDM lyophilisée et stable au stockage selon la revendication 1, caractérisé en ce que le sel de métal alcalin est un chlorure de métal alcalin.

3. Procédé de préparation d'une composition de MDM lyophilisée et stable au stockage selon la revendication 1, caractérisé en ce que le sel de métal alcalin est le chlorure de potassium.

4. Procédé de préparation d'une composition de MDM lyophilisée et stable au stockage selon la revendication 1, caractérisé en ce que le sel de métal alcalin est le chlorure de sodium.

5. Procédé de préparation d'une composition de MDM lyophilisée et stable au stockage selon la revendication 1, caractérisé en ce que la substance tampon du pH est le phosphate de sodium.

6. Procédé de préparation d'une composition de MDM lyophilisée et stable au stockage selon la revendication 1, caractérisé en ce que la solution aqueuse obtenue de l'opération (d)(i) est maintenue à une température d'environ 0°C à 5°C pendant une durée maximale de 15 heures avant d'effectuer l'opération (e) en vue d'inhiber une épimérisation de plus de 5,5% de l'acide stéréo-isomère (5R,6R)-benzyl-D-pénicilloïque.

7. Procédé de préparation d'une composition de MDM lyophilisée et stable au stockage selon la revendication 1, caractérisé en ce qu'il comprend également les opérations d'interruption du vide dans les récipients à l'aide de nitrogène filtré et séché.

8. Procédé de préparation d'une composition de MDM lyophilisée et stable au stockage selon la revendication 1, caractérisé également en ce que les récipients déshydratés sont fermés hermétiquement immédiatement après interruption du vide.

9. Procédé de préparation d'une composition de MDM lyophilisée et stable au stockage selon la revendication 1, caractérisé en ce que la lyophilisation de la solution congelée est effectuée en une première phase durant environ 8 à 13 heures à une température d'environ -45°C à -15°C, sous un vide d'environ 15 à 30 µm, puis en une seconde phase durant environ 7 à 14 heures à une température d'environ 0°C à 5°C, sous un vide d'environ 15 à 30 µm, la durée totale de dessiccation étant d'environ 20 à 22 heures.
